# EUROPEAN PATENT APPLICATION

(11) **EP 3 725 216 A1**
(43) Date of publication of application: **21.10.2020**
(21) Application number: 19170408.9
(22) Date of filing: 19.04.2019
(51) Int. Cl.: A61B 5/024, A61B 5/11

(54) **WEARABLE SENSOR**

(71) Applicant: Koninklijke Philips N.V., 5656 AG Eindhoven (NL)
(72) Inventor: TE VELDE, Mart Kornelis-Jan, 5656 AE Eindhoven (NL); TOONEN, Stefan Johannes Hendrikus, 5656 AE Eindhoven (NL); BHAT, Ravindra, 5656 AE Eindhoven (NL)
(74) Representative: de Haan, Poul Erik

(57) **Abstract**

The invention proposes a wearable sensing device (100) comprising a sensing element (180) to sense physiological signs of a user wearing the device, electrical circuitry (170-172), air- or oxygen-dependent battery(ies) (110) comprising an air-inlet surface (111). The wearable sensing device further comprises a housing (130) of the sensing element (180), the circuitry (170-172) and the at least one battery (110), all functionally electrically arranged together.

The housing (130) comprises casing elements (131, 131') arranged to lock the at least one battery (110) in a predetermined locking position. The housing (130) further comprises a base (136) and a cover (135) mounted to the base (136) in a manner and in such a way that an internal gap (132) extends between said battery air-inlet surface (111) and the cover (135). The base (136) and the cover (135) are further arranged such that said air-inlet surface (111) has an access to ambient air.

The invention further proposes a wearable sensing device (100') arranged before use with sealing tabs (140) to protect said air-inlet surfaces (111) from air contact.

## Description

### FIELD OF THE INVENTION

The disclosure relates to autonomous wearable devices, methods and systems for detecting physiological signs, such as for instance vital signs and/or activities, of a user wearing this device.

### BACKGROUND OF THE INVENTION

Wearable sensors are being developed for in- and out- of hospital monitoring of patients. They usually measure physiological signs, such as vital signs (e.g. heart rate, blood pressure, respiratory rate, biomarkers) and/or activities (e.g. running, walking, laying, cycling, sleeping etc.).

Such wearable sensors must be autonomous and easy to use by nurses and users at home.

Batteries are typically used for providing the autonomous factor of the energy supply.

In the recent years, wearable sensors with zinc-air batteries have been developed, these batteries can run sensors for a month and handle the peak current that is needed for data transmission with a wireless long range radio transmitter. Zinc air batteries have furthermore high capacity to volume ratio and are environmental friendly compared to other typical coin cell batteries (e.g. Lithium, Alkaline, ...).

Now zinc-air batteries work in contact with oxygen, and removable battery seals are thus typically used to protect them from power discharge before usage. Such air-zinc batteries with battery seals are known in hearing aids. The user removes then the seal and places the battery in a holder in the device. These hearing aids are typically not waterproof.

A known solution to get airflow in a device while keeping it waterproof is the use of a watertight membrane where air can pass through. Now this kind of solution results in a few drawbacks in a nursery site or at home:
the nurse or the user needs to make sure that batteries are available;
handling multiple parts (2 batteries + 1 device) to prepare the patch is not favorable.

This solution requires a certain device volume to ensure that the user/nurse places the battery correctly with a proper spring force without damaging the device and a more cumbersome device is not favorable for a wear comfort.

### SUMMARY OF THE INVENTION

The present invention improves the previous wearable sensing devices by proposing:
a wearable sensing device ready for use according to claim 1, as a first embodiment, and
a wearable sensing device before use according to claim 2, as a second embodiment.

Said first embodiment of wearable sensing device for use or ready to use, comprises:
one sensing element to sense physiological signs of a user wearing the device;
electrical circuitry to process signals from the sensing element;
at least one air- or oxygen-dependent battery adapted to power the sensing element and the circuitry, comprising an air-inlet surface;
a housing of the sensing element, the circuitry and the at least one battery which are functionally electrically arranged together;
wherein the housing comprises:
casing elements arranged to lock the at least one battery in a predetermined locking position; a base; and
a cover mounted to the base in a watertight manner and in such a way that an internal gap extends between the air-inlet surface of at least one battery and the cover, the internal gap being formed by an aperture initially provided in the cover and/or in the base before montage;
wherein the base and the cover are further arranged such that said air-inlet surface has an access to ambient air.

In said first embodiment representing the wearable sensing device ready to use, the battery(ies) is (are) locked. "Locking" an object inside a system means mechanical holding, without any soldering, of this object by surrounding holding elements provided in the system in a sufficiently robust manner that the object is not significantly moved inside and with respect to the surrounding holding elements even if the system is moved, displaced or shaked, and hardly moved if the system experiences an impact (e.g. a fall or is submitted to a violent shock) as long as any elements of the system is not broken. In particular, a locking may be typically successful if said surrounding elements apply sufficiently high, opposite and inward mechanical forces on opposite sides of the object. According to this first embodiment, the battery(ies) is (are) then locked (and so mechanically and fixedly hold) by the housing in such a way that it cannot significantly be moved inside and with respect to the housing if the device is moved, displaced or shaked, and hardly moved if the device falls or is submitted to a violent shock as long as any element of the device 100 is not broken. The locking of the battery(ies) by the housing per se allows the batteries to be held in a fixed position by mechanical means independent from the electrical connections, and therefore preventing creations of any strains or load at the electrical interface(s) between the batteries and the external electrical circuitry of the wearable sensing device - which might be of importance if the battery(ies) is (are) for instance soldered to the electrical circuitry.

Furthermore such locking of the battery at a fixed position is key to position with accuracy the battery with respect to the electrical circuitry, improving therefore the electrical connection between the two.

In particular, this accurate positioning helps and allows an electrical connection made via a simple mechanical contact between the electrical contact(s) of the battery(ies) and the electrical contact(s) of the external electrical circuitry. This can therefore prevent from soldering the battery to the electrical circuitry, which significantly simplifies the manufacturing and the costs of the wearable sensing device.

The internal gap facing the air-inlet surface comprises a gap that has been typically left after having removed an air-sealing element which was protecting the air-inlet surface of the battery before mounting the cover to the base.

Moreover this internal gap provides a free space above the air-inlet surface of the battery facilitating air access and helping thus the battery to operate in a an optimal way.

Furthermore the wearable sensing device provides such internal gap, freed after removal of said sealing element while keeping or ensuring a watertight montage.

The cover of the aperture(s) provided in the housing of the wearable sensing device, is mounted to the base of the housing in a watertight and air permeable manner. Therefore the cover is not integral with the other elements of the housing, which allows then a montage of the cover to the other elements of the housing just before using the wearable sensing device. In particular, this cover allows a water-tight closing of this aperture just after an air sealing element (as above-defined) is removed from the aperture, protecting then the battery from the environment while leaving an air access to the battery.

The present invention proposes, according to a second embodiment, a wearable sensing device comprising:
one sensing element to sense physiological signs of a user wearing the device;
electrical circuitry process signals from the sensing element;
at least one air- or oxygen-dependent battery adapted to power the sensing element and the circuitry comprising an air-inlet surface;
a housing of the sensing element, the circuitry and the at least one battery which are functionally electrically arranged together;
a sealing tab comprising a battery sealing element which air-tightly covers directly the air-inlet surface of at least one battery, and an extended tab extending from the sealing element and adapted to be manually actionable;
wherein the housing comprises casing elements arranged to lock the at least one battery in a predetermined locking position; and
wherein the housing and the sealing tab are arranged such that the sealing tab is manually removed by peeling it away from the air-inlet surface of the battery and the housing is adapted to have a watertight configuration after the removal of the sealing tab.

This second embodiment represents the wearable sensing device before usage, the sealing tab protecting the air-inlet surface of the battery from being in contact with air, and then preventing the battery from power discharge.

The locking of the battery(ies) by the housing per se allows the batteries to be held in a fixed position mainly by mechanical means independent from the electrical connections, and therefore preventing creations of any strains or load at the electrical interface between the batteries and the external electrical circuitry of the wearable sensing device - which might have some importance if the battery(ies) is (are) for instance soldered to the electrical circuitry.

Furthermore such locking of the battery at a fixed position is key to position with accuracy the battery with respect to the electrical circuitry, improving therefore the electrical connection between the two.

In particular, this accurate positioning helps and allows an electrical connection thanks to a simple mechanical contact between the electrical contact(s) of the battery and the electrical contact(s) of the external electrical circuitry. This can therefore prevent from soldering the battery to the electrical circuitry, which significantly simplifies the manufacturing and the costs of the wearable sensing device.

Furthermore this locking of the battery in the housing allows the sealing tab to be removed from the housing without moving the battery in or out from the housing, the housing retaining the battery via its casing elements. This may be particularly useful since it is usual that the sealing tab is stuck to or pressed against the air-inlet surface of the battery(ies), and tends therefore to pull the battery out from the housing when the user attempts to remove it, especially if the sealing tab is removed in a direction at least partly normal to the air-inlet surface of the battery.

Furthermore this second embodiment warrantied a good air-tight sealing of the battery(ies) before usage since the sealing element is directly in contact with the air-inlet surface of the battery. This prevents thus having any intermediary material which could reduce the air-tightness of the battery before usage. Furthermore it avoids any damages of any intermediary layers that would have been sandwiched between the battery and the sealing element, after the sealing tab is removed, which could thus hamper the use of the wearable sensing device.

Furthermore, the housing and the sealing tab are arranged such that the sealing tab is manually removed by peeling it away from the air-inlet surface of the battery and the housing is watertight before and after the removal of the sealing tab.

"Peeling away" means exerting a force having a non-negligible component in a direction normal to and away from the air-inlet surface of the battery. By peeling the tab away from the air-inlet surface of the battery, the force needed to remove the sealing tab is significantly less important than if one tries to remove the tab by exerting a sheering force (i.e. a force mainly exerted in a direction parallel to the air-inlet surface of the battery). To succeed in peeling the sealing tab away from the air-inlet surface of the battery, the housing must be configured to allow this manual pulling of the sealing tab away from the battery. In particular, the housing may exhibit, before usage, a free space above the sealing tab (i.e. a volume extending from the side of the sealing tab opposite to the side of the sealing tab facing the air-inlet surface of the battery) sufficiently large to allow fingers of a human hand to catch the sealing tab and peel it away. Furthermore, the locking of the battery in the housing, thanks to the casing elements of the housing, warrants that this peeling of the sealing tab away from the air-inlet surface of the battery does not move the battery in the housing, as above-mentioned.

In a particular embodiment of this second embodiment, a first part of the extended tab of at least one sealing tab is folded in the housing and a second part of the extended tab extends beyond the housing through an opening of the housing.

This folded sealing tab allows having a part of the sealing tab extending out from a side of the housing (e.g. through a slot thereof) while keeping the possibility for the sealing tab to be removed by peeling it away from the air-inlet surface of the battery - which is easier to do than exerting a sheering force as above-mentioned. Indeed, if the sealing tab would have not been folded inside the housing (but instead, would have been directly extended from the air-inlet surface of the battery to the slot of the housing) the force that would have been needed to remove the sealing tab would have been mainly a sheering force. Moreover the folding allows having a longer tab once unfolded, which facilitates the unsealing thanks to a higher leverage. Furthermore, this configuration allows a housing having a cover extending over the top of the air-inlet battery surface, to protect the battery and more generally the inner volume of the housing (enclosing the electrical circuitry, the sensing element etc.). Briefly, this configuration allows a protection of the components in the housing while exhibiting an easy activation of the battery (by peeling the sealing tab away from the air-inlet surface of the battery).

In an even more particular embodiment, a flexible sealing component is provided in the opening to provide a watertight sealing after the sealing tab is removed, and allows a watertight protection of the housing even after having removed the sealing tab through the opening.

In a particular embodiment of said second embodiment, the wearable sensing device comprises a plurality of batteries and a plurality of sealing tabs, and wherein the sealing tabs are provided with a common tongue extending outside the housing. A common tongue allows an activation of all the batteries in one shot, quicker and with higher force.

In a particular embodiment of both said second embodiment, the housing comprises a base and a cover, the cover being mounted or to be mounted to the base in a watertight manner, the cover and/or the base being provided with an aperture that will form an internal gap extending between the air-inlet surface of at least one battery and the cover once the sealing tab is removed and the cover is mounted to the base, and optionally the sealing element extends on the air-inlet surface of the battery across or beneath the aperture.

This embodiment may allow the protection of the air-sensing surface of the battery once the sealing tab is removed, and more generally the protection of any and all components lodged inside the housing, from humidity and environment.

The internal gap facing the air-inlet surface comprises a gap that has been left after having removed the sealing element from the air-inlet surface of the battery.

Moreover this internal gap provides a free space above the air-inlet surface of the battery facilitating air access and helping thus the battery to operate in a an optimal way.

Furthermore the wearable sensing device provides such internal gap, freed after removal of said sealing element while keeping or ensuring a watertight montage.

In a particular embodiment of said first or second embodiment, the aperture or the internal gap facing the air-inlet surface of a battery is sufficiently narrow in at least one direction to prevent the corresponding battery to leave out the housing. This narrow aperture of the housing, facing the air-inlet surface of the battery, allows on one hand air to access the battery air-inlet surface and on the other hand to participate in the locking of the battery(ies) in the housing.

Furthermore, this narrow aperture may be useful to retain by abutment the battery(ies) when the sealing tab, usually stuck to or pressed against the air-inlet surface of the battery(ies), is removed from this aperture just before usage. The locking of the battery(ies) at a predetermined position in the housing is therefore guaranteed by this narrow aperture also during the sealing tab removal, the narrow aperture retaining the battery(ies) when the sealing tab is removed in a direction at least partly normal to the aperture (i.e. during the "peeling away" action).

Moreover this aperture may be designed to have a complementary shape to the shape of the sealing element, such that the sealing element extends across or just beneath the aperture, optimizing the low volume of the wearable sensing device. This aperture, thus surrounding the sealing element, may further protect the sealing element from being partly removed or the sealing to be less good before usage, and so the battery to be at least partly discharged before usage. Alternatively, the sealing element may extend above the aperture, or be in according to any combination thereof (i.e. beneath, across and/or above the aperture).

In a particular embodiment, the cover is provided with semi-permeable membrane (i.e. being water-tight and air-permeable).

In another particular embodiment, the cover is arranged to be slid onto the base to a fixed and watertight position, and wherein the base and/or the cover further comprise attachment elements to secure an attachment of the cover to the base at the fixed and watertight position.

This configuration provides a particularly robust wearable sensing device, especially if the cover is made of a rigid material.

In an alternative particular embodiment, the cover is arranged to be foldable on a top side of the base provided with the at least one aperture to a fixed and watertight position, and wherein the base and/or the cover further comprise attaching elements to secure an attachment of the cover to the base at the fixed and watertight position. This alternative particular embodiment proposes a wearable sensing device which could be made in a single piece, the cover being fixed to the base at the folding axis or having a non-foldable part fixed to or integral with the base. This is easier to use, easy to understand, and there is less risk of losing a part of the device.

Also the wearable sensing device may be packed with the cover in its folded state, allowing then a smaller packaging, which means: storage, distribution and shipment of the packed device less costly, more robust, more convenient, useful and a device visually more attractive in shops.

Optionally, the extended tab of at least one sealing tab is at least partly folded when the cover is in an unfolded position such that the force for peeling the tab away from the air-inlet surface of the battery(ies) is minimized since it can be exerted in a direction normal to the air-inlet surface. Additionally, its shape (folded tab) guides the user to peeling it way in a right way.

Also the wearable sensing device may be packed with the cover in its folded state, with the sealing tab in its folded state too, allowing then a smaller packaging, which means: storage, distribution and shipment of the packed device less costly, more robust, more convenient, useful and a device visually more attractive in shops.

In a particular embodiment of both said first and the second embodiments, wherein the aperture is provided in the housing facing the air-inlet surface of the battery, at least one portion of the housing provided on edges of the aperture is arranged to be elastically deformable to allow the battery to be positioned into the housing.

This particular embodiment allows the user, the nurse, or the manufacturer to insert the battery in the housing, in its locked position. This embodiment may further be arranged to prevent it from being removed. This embodiment is particularly interesting for single use of the wearable sensing device. It also prevents user to manipulate batteries and to damage the wearable sensing device if the batteries are already locked into the device before it is acquired / purchased by the final user - those batteries may be for instance positioned during manufacturing.

In another particular embodiment of both said first and the second embodiments, casing elements of the housing are provided with inner opposite walls fitting to at least one battery such that opposite sides of the battery are sufficiently pressed by opposite inner walls to hold the battery in the housing. That could be before or after the cover is mounted to the base.

This embodiment allows an easy positioning of the battery inside the housing, which may have multiple advantages, including an easier, faster and less costly montage of the wearable sensing device, since a single push of the battery inside the housing is needed for this montage, and optionally for its electrical connection to the other electrical circuitry lodged in the housing.

In another particular embodiment of both said first and the second embodiments, the wearable sensing device is wireless. In particular it further comprises a communication circuitry arranged to wirelessly communicate sensing data, optionally processed by the electrical circuitry, to a separated receiver, and optionally to further wirelessly receive from a separated transmitter, parameters or instructions for tuning or controlling the wearable sensing device.

In another particular embodiment of both said first and the second embodiments, the casing elements of the housing comprises a circuit board on which the electrical circuitry is provided, the circuit board being positioned at an inner bottom side of the housing, and wherein the at least one battery is positioned on and electrically connected to the PCB and extending between the inner bottom side and the top side of the housing.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 depicts a cross-section of a wearable sensing device in operation according to a first embodiment;
Fig. 2 is a cross-section view schematically showing the energy power source section of a wearable sensing device before use, according to a second embodiment;
Fig. 3 is a cross-section view schematically showing the energy power source section of a wearable sensing device before use, according to a third embodiment;
Fig. 4 is a cross-section view schematically showing the energy power source section of a wearable sensing device before use, according to a fourth embodiment;
Fig. 5a and 5b are cross-section views schematically showing the energy power source section of a wearable sensing device before and after use (respectively), according to a fifth embodiment;
Fig. 6a and 6b are perspective views showing a wearable sensing device before and after use (respectively), according to a sixth embodiment;
Fig. 7a to 7d are perspective views showing the same wearable sensing device in use (according to successive steps), according to a seventh embodiment;
Fig. 8a is an exploded view showing a part of a wearable sensing device before use, according to an eighth embodiment, this eighth embodiment comprising a top cover 135 which appears transparent in this Figure 8a for illustration purpose (it may be transparent, opaque or semi-transparent) ;
Fig. 8b and 8c are perspective views showing the wearable sensing device before and after use (respectively), according to the eighth embodiment; and
Fig. 9 is a cross-section view schematically showing the energy power source section of the wearable sensing device before use, according to the eighth embodiment.

### DETAILED DESCRIPTION OF EMBODIMENTS

As noted above, there is proposed herein an improved device for quantitatively sensing physiological signs. The device can be configured to be worn by a subject. Thus, the device can be a wearable device.

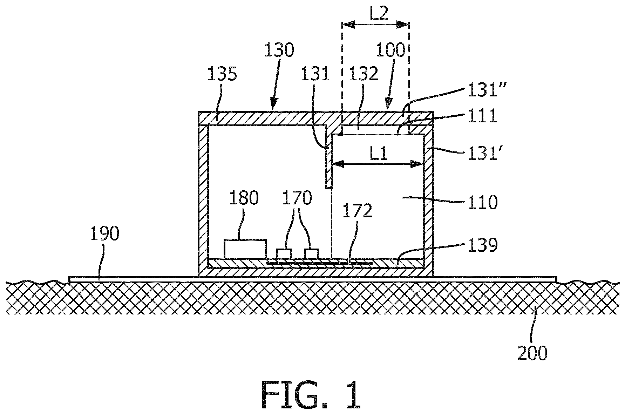

Fig.1 illustrates a wearable sensing device 100 positioned on a location (e.g. skin) 200 of a body of a user. The wearable sensing device 100 comprises:
a sensing element 180 to sense physiological signs of the user wearing the device 100;
electrical circuitry 170-172 to process signals from the sensing element 180;
at least one air- or oxygen-dependent battery 110 adapted to power the sensing element 180 and the circuitry 170-172, and comprising an air-inlet surface 111;
a housing 130 of the sensing element 180, the circuitry 170-172 and the at least one battery 110. The three last components are functionally electrically arranged together.

In some embodiments, the device 100 may further comprise a user interface 190, such as a strap (or band) for placement on and/or for adhering to the skin or around a part of the body of the subject, such that the device can be worn at any suitable location. In some embodiments, an adhesive layer is provided on the underside of the device 100 for fixation of the device 100 to the skin of the subject. In case the sensing element 180 necessitates a particular interaction with the user 200, then the interface may be arranged to facilitate such interaction while keeping a good user interface and/or adhesivity. For instance, the interface 190 may be provided with optics if the sensing element 180 comprises optical sensors, or with an aperture for lodging the sensing element 180, or with a flexible membrane for a sensing element 180 arranged to sense movement of or through the skin or to transmit ultrasound, or being at least partly constituted of gel if the sensing element 180 comprises an electrode or ultrasound sensor that requires a gel interface, or being arranged to collect a biological fluid (e.g. sweat, blood) for a sensing element 180 dedicated to sense information from such fluid.

The sensing element 180 for sensing or detecting physiological signs of the user can be of any type. Sensing element 180 may comprise accelerometer to sense physical activity (e.g. rest, walking, running, ambulatory, posture - sitting, standing-up, laying, etc.) and/or vital signs (e.g. respiratory rate - "RR", heart rate - ""HR", blood pressure - "BP"...). Sensing element 180 may comprise dry or wet electrode for e.g. sensing vital signs (HR, RR, BP, etc.). Sensing element 180 may comprise optical sensor such as for instance photoplethysmography ("PPG") sensor for sensing e.g. vital signs (HR, RR, BP, oxygen information in blood, biomarkers information in blood, etc.). Sensing element 180 may comprise ultrasound sensor to monitor physiological signs of the user sensitive to ultrasound. Sensing element 180 may comprise fluidic sensor to sense physiological signs in the collected fluid (sweat, blood, etc.). Sensing element 180 may comprise a plurality of sensors, such as for instance any combination of the above-mentioned ones.

Electrical circuitry 170-172 is further provided to process signals from the sensing element(s) 180.

In particular electrical and/or electronic components 170 may be provided to process the data/signals for a next use thereof and to store data in memory before and/or after data/signal processing. Such data processing may be arranged to retrieve raw data from the signals received from the sensing element 180 and to modify such raw data in view of a next data processing or of extracting some meaningful information for a dedicated application or of performing a CAN or CNA. In an even more particular embodiment, the electrical components 170 comprise an antenna to wirelessly communicate data to at least one remote receiver (e.g. a computer or another remote monitoring or display or cloud, etc.), and components to prepare and/or modify the data to be wirelessly communicated. For instance these components 170 may comprise a chip that stores instructions for packing and/or encoding the data in blocks of data in view of their wireless transmission, according to a standardized or non-standardized protocol (e.g. LoRA, WiFi, Bluetooth, etc.). Optionally, the some electrical components can be further or additionally provided with means for receiving data / instructions from an external controller / transmitter, and process them. These data or instructions may be used to tune, change or reset the electronic functionalities or parameters of electronic components used in the wearable sensing device 100.

Electrical circuitry 170-172 also typically comprises wires or circuits 172 or the like to make the different electrical components of the wearable sensing device 100 interconnected together in an operational manner, and/or comprises some components to protect or secure all the electrical system of this device 100. Such wires or circuits 172 may be provided or printed in or onto an electrically insulating board (such as a plastic circuit board - "PCB") 139.

The device 100 further comprises at least one air- or oxygen-dependent battery 110 adapted to power the sensing element 180 and the circuitry 170-172. This at least one battery 110 comprises an air-inlet surface 111. Such air- or oxygen-dependent battery 110 operates thanks to the air or oxygen provided through the air-inlet surface 111. In a particular embodiment, the invention uses battery 110 such that air or oxygen is used as a reactant of a chemical reaction with a chemical component already stored in the cell of the battery. In an even more particular embodiment, the invention uses zinc-air battery(ies), also called "zinc-air fuel cells", which are non-rechargeable, electrochemical batteries powered by the oxidation of zinc with oxygen from the air. These batteries have very high energy densities and are relatively inexpensive to produce. However, instead of packing the necessary materials (ingredients) inside the cell as other batteries, air- or oxygen-dependent batteries 110 get one of their main reactants, namely, oxygen or air, from the outside air. Using a reactant from the air reduces the size and space requirements of the battery, and thus reduces the dimensions and weight of the device retaining the battery. Also, air- or oxygen-dependent batteries 110 contain no toxic compounds and are neither overly reactive nor flammable. Thus, zinc-air batteries can be recycled and safely disposed of. Other advantages of these batteries can be found in the literature. Now in case these batteries 110 have not enough power, electrical arrangement that includes one or more capacitors can be added in the components 170, to provide sufficient charge to power, at least initially.

Additionally, the device 100 comprises a housing 130 which lodges the different components (battery(ies) 110, electrical circuitry 170-172 and sensing element(s) 180). The housing 130 is typically made of rigid material(s) (e.g. of a polymeric material), arranged to absorb most of mechanical shocks. Housing 130 is further provided with inner casing elements 131-131'-131" designed to lock the at least one battery 110 in a predetermined locking position. In a particular case, the insulating board 139, optionally bearing some printed or etched circuitry 172, is part of the housing 130 and is used as a casing element together with the opposite casing element 131", and optionally with other lateral casing elements 131 and 131'. "Locking" means that the battery 110 is mechanically and fixedly hold in the device 100 in such a way that it cannot significantly be moved inside and with respect to the housing 130 if the device 100 is moved, displaced or shaked, and hardly moved if the device falls or is submitted to a violent shock as long as any element of the device 100 is not broken. In other words, the housing 130 is arranged such that some casing elements (131-131'-131"-139) apply opposite mechanical pressures on opposite sides of the battery 110 sufficiently high to fixedly hold the battery 110.

Furthermore the casing element 131" may comprise an aperture 132 facing the air-inlet surface 111 of the battery 110. A width "L2" of this aperture 132 is sufficiently narrow in a direction to prevent the battery 110 to go through this aperture 132. In particular, the battery 110 as locked has a width "L1", as measured along a direction parallel to the measuring direction of said width "L2", lower than "L2". As a consequence, the battery 110 cannot go through the aperture 132. In a particular configuration, some edges of the aperture 132 constitute of abutments to the battery 110 in a direction away from and normal to the air-inlet surface 111 of the battery 110.

The housing 130 further comprises a cover 135 on at least the aperture(s) 132 in a watertight and air permeable manner. This cover 135 may be integral with other or all other parts of the housing. Alternatively the cover 135 may be mounted to other or all other parts of the housing 130 in a watertight and air permeable manner, thanks for instance to any kind of water sealings and/or adhesive and/or pressurized means that can be used at the interface between the cover 135 and other adjacent parts of the housing 130. This watertight interface can be arranged to leave an air access. Alternatively or in combination, at least one semi-permable membrane (i.e. a watertight and air-permeable membrane) may also be provided through the housing 130, in particular through the cover 135, to ensure a good air access to the air-inlet surface 111 of the battery 110. One can note that the cover 135 may further provide a large surface to the device 100 on which a marking can be applied such as e.g. a product label.

Generally speaking, the whole housing 110 is watertight, while still providing an air access to the air-inlet surface 111 of the battery(ies) 110.

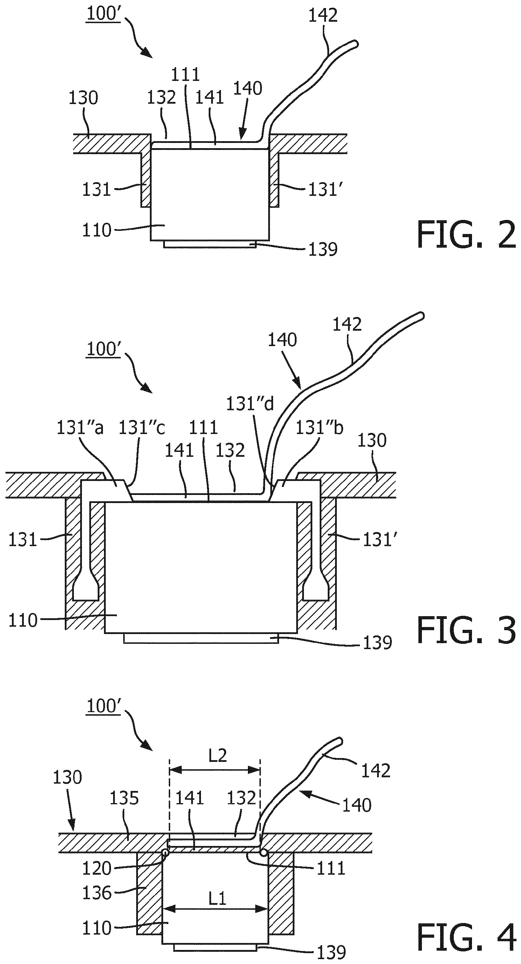

Figs 2 to 4 show three different embodiments of wearable sensing device 100' before usage. For sake of simplicity these figures depict only power supply sections of the wearable sensing device 110'.

As above-indicated, the air- and oxygen-dependent batteries 130 are only active when their air-inlet surfaces 111 (typically air or oxygen cathodes) have access to air. In order to preserve the batteries 110, some battery seal tabs 140 are then provided above the air-inlet surfaces 111 for preventing air to access those surfaces 111. This is of course particularly useful if batteries 110 need to be stored and shipped, separately or with the remaining part of the device 100'.

A sealing tab 140 comprises a battery sealing element 141 which is arranged to air-tightly cover the air-inlet surface 111 of a battery 110. Such a sealing element 141 may be stuck (via adhesive material around the air-inlet surface 111 of the battery 110 and/or being pressurized on the battery 110). The sealing tab 140 may further comprise an extended tab 142 or a tongue, extending from the sealing element 141, and adapted to be manually actionable.

In a preferred embodiment, the sealing element 141 of the sealing tab 140 covers directly the air-inlet surface 111 of a battery 110, i.e. there is no material (except perhaps some usual molecules or dusts) between the sealing element 141 and the air-inlet surface 111. The sealing element 141 may extend at least partly over the aperture 132 of the housing. This particular embodiment has the advantage that the sealing element 141 does not constitute an additional layer in the device 100' - and so the volume of the device 100' is minimized. Moreover, such aperture 132 may also be a further mechanical protection of the sealing element 141 from it being at least partly removed from the air-inlet surface 111 of the battery 110 - which would lead to a discharge of the battery 110.

Furthermore the housing 130 and the sealing tab 140 are arranged in such a way that the sealing tab 140 is manually removed by peeling it away from the air-inlet surface 111 of the battery 110. Indeed, as depicted by Figs 2 to 4, the location of the sealing element 141 inside a "hole" - i.e. aperture 132 - and/or in a free space provided on the side of the sealing element 141 opposite the battery 110, makes this removal of the sealing tab 140 intuitive and possible. As above-explained, the peeling of the sealing element 141 away from the air-inlet surface 111 of the battery 110 is easier to do in comparison with a sheering force exerted on the sealing tab 140. Furthermore, since the sealing element 141 is directly applied (typically stuck to) the air-inlet surface 111 of the battery 110, its removal leads to a significant pulling force onto the battery 110 in a direction normal and away from the air-inlet surface 111 of the battery 110. One might provide an intermediate, elastic and breathable material between the sealing tab 140 and the air-inlet surface 111 of the battery 110 to damp the pulling force exerted onto the battery 110. But then this additional material may increase the device 100' costs and may bring additional issues. Now, according to the invention, a retained solution was to provide the housing 130 with casing elements 131-131', 131"a-131"b-139 as depicted in Figs. 2 to 4, to keep the battery 110 locked in its locking position even when the sealing tab 140 is manually removed - and thus exerts a pulling force onto the battery 110.

Fig. 2 depicts casing elements 131 and 131' of the housing 130 provided with inner opposite walls fitting the battery 110 such that opposite sides of the battery 110 are sufficiently pressed by these opposite inner walls to hold the battery 110 in the housing 130 in the locking position, and in particular when the sealing tab 140 is removed. Typically, the battery 110 has been introduced into the housing 130, between the casing elements 131-131', by exerting a significant force, the inner walls 131-131' of the housing 130 moving then slightly apart from each other upon this mechanical action to reach the desired fit at a locking position. Some slots/ridges or other means might be provided on the sides of the battery 110 and/or on the sides of the inner walls 131 -131' to guide the battery 110 between the inner walls 131-131' until the desired locking position. Mechanical stops may also be further provided on the sides of the battery 110 and/or on the sides of the inner walls 131-131' to define the end of the guiding of the battery 110 between the inner walls 131-131' and thus to define the desired locking position of the battery 110 inside the housing 130.

Fig. 3 depicts other types of casing elements 131a" and 131"b of the housing 130. Such casing elements 131a"-131"b are respectively edges of the aperture 132 (facing the air-inlet surface 111 of the battery 110) which are arranged to be manually and elastically deformable to allow the battery 110 to be positioned into the housing 130 at the locking position through the aperture 132 (which is then wider when the edges 131a"-131"b are deformed). In order to facilitate the introduction of the battery 110 into the housing 130, the terminal parts 131c"-131"d (i.e. the parts adjacent to the aperture 132) of the respective edges 131a"-131"b are beveled to exhibit end surfaces 131c"-131"d diverging away from each other and from the air-inlet surface 111 of the battery 110. On the contrary these beveled terminal parts 131c"-131"d will prevent (or make hard) the removal of the battery 110 from the housing 130. Preferably a width of the aperture 132 (if the edges 131 a"-131"b are not deformed) is sufficiently narrow to prevent the battery 110 to go through this aperture 132. In particular, the battery 110 as locked has a width (as measured along a direction parallel to the measuring direction of said width of the aperture 132), lower than said width of the aperture 132. The battery 110 cannot therefore go through the aperture 132. In this particular configuration, the edges 131a"-131"b of the aperture 132 are abutments to the battery 110 in a direction away from and normal to the air-inlet surface 111 of the battery 110. Optionally the housing 130 is provided with another casing element (which may be the insulating board) 139 that may be fixed and positioned at a distance with respect to the aperture 132 which corresponds to the height of the battery 110: the battery 110 is then locked between the edges 131a"-131"b and this casing element 139. Optionally, some other casing elements 131-131' may be provided on the sides of the battery (i.e. along the height of the battery 110) to provide a compartment and a further guidance when the battery 110 is introduced. In a particular case, these casing elements 131-131' are inner opposite walls fitting to the battery 110 such that the opposite sides of the battery 110 are sufficiently pressed by these opposite inner walls to hold the battery 110 in the housing 130 in the locking position, and in particular when the sealing tab 140 is removed - such as the above-embodiment depicted by Fig. 2.

Fig. 4 shows a similar embodiment as the one of Fig. 3, except that the edges of the narrow aperture 132 are not beveled. As a further difference from the device 100' of Fig. 3, the housing 130 is in this embodiment made of two parts, a cover 135 and a base 136, watertightlymonted one to the other, via e.g. some sealing components 120, once the sealing tab 140 is removed. Furthermore the aperture 132 is formed by a recess provided on the inner side of the cover 135, the recess 132 having a width "L2" sufficiently narrow in a direction to prevent the battery 110 to go through this aperture 132 (in particular, the battery 110 as locked in the housing 130 has a width "L1", as measured along a direction parallel to the measuring direction of said width "L2") lower than "L2": the battery is then locked into the housing 130 by abutment to the edges of the recess 132.

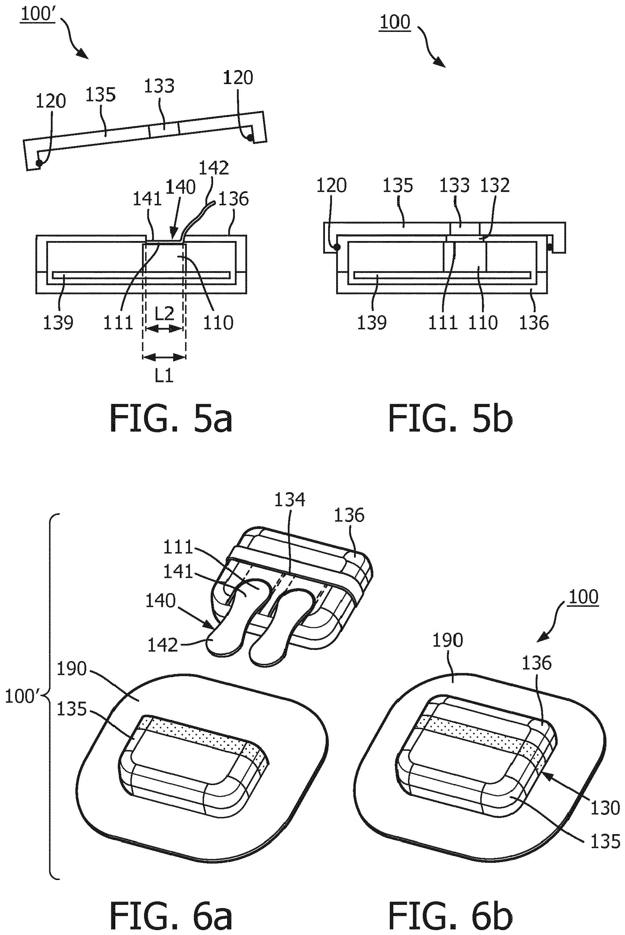

Fig. 5a and 5b schematically illustrate respectively a wearable sensing device 100' before use and a wearable sensing device 100 in operation or ready to use. Similarly to the embodiment of Fig.4, the aperture 132 is narrower than a width of the battery 110 and the housing 130 is made of two parts, a cover 135 and a base 136, watertightly monted one to the other, via e.g. some sealing components 120, once the sealing tab 140 is removed. The aperture 132 is formed by a recess 132 provided on an inner side of the cover 135, or alternatively or in combination, by an aperture provided on top of the base 136, facing the air-inlet surface 111 of the battery 110. In this embodiment, at least one semi-permable membrane 133 (i.e. a membrane 133 which is watertight and air permeable) is provided through the cover 135. The membrane 133 may face the air-inlet sensing surface 111 of each battery 110, or maybe located elsewhere - as long as an air access to the air-inlet sensing surface 111 is provided between the cover 135 and the base 136, extending from the membrane 133 to the air-inlet surface 111). It is to be noted that the battery(ies) 110 is locked in this embodiment too, even if this illustrative and schematic drawing does not really show it: that could be done for instance by fixing the insulating board 139 in the housing 130 as a component of the housing 130, and/or by providing any kinds of casing elements such as for instance the ones disclosed in this document.

Fig. 6a and 6b show respectively a wearable sensing device 100' before use and a wearable sensing device 100 in operation or ready to use, comprising two air- or oxygene dependent batteries (not shown here). Similarly to the embodiment of Figs. 4 and 5, the housing 130 is made of two parts, a cover 135 and a base 136, watertightly monted one to the other once the sealing tabs 140 are removed. In this particular embodiment the cover 135 and the base 136 are encasable one to the other in a complementary manner, and are provided with an interface 134 arranged to mount one to the other in a fixed (optionally in a non-reversible) and watertight manner. Arrangement of the montage may use complementary mechanical connecting means (e.g. a pair of ridge/groove along the interface 134, one provided on the cover 135 the other one on the base 136) that are watertight by their own, without any need of additional water sealing element. Now additional water sealing element(s) (not shown) may be provided at the interface 132 and/or inside the housing 130 beneath the interface 132, to ensure a good watertightness. In this embodiment, the cover 135 may be a rigid casing which would host at least a portion of the base 136, and may be slid onto the base 136 to cover the unsealed air-sensing surfaces 111 of the batteries. A body interface 190 (patch, paster, etc.) may be provided on the cover 135 or on the base 136. A narrow aperture (not really shown here) can be provided in the housing 130 (i.e. in the cover 135 and/or in the base 136) above each air-inlet sensing 111 of each one of the two batteries once the sealing tabs 140 are removed, some other casing elements (not shown) may be provided too to the housing to lock the batteries in a secured way inside the housing 110, and one (or more) semi-permeable membrane (not shown) may be provided through the cover 136. Other option as above-indicated may also be provided to this particular embodiment. In an even more particular embodiment, the narrow aperture (not really shown here) is provided in the base 136 above each air-inlet sensing 111 of each one of the two batteries once the sealing tabs 140 are removed, and the cover 135 is also used to help locking the batteries in the base 136.

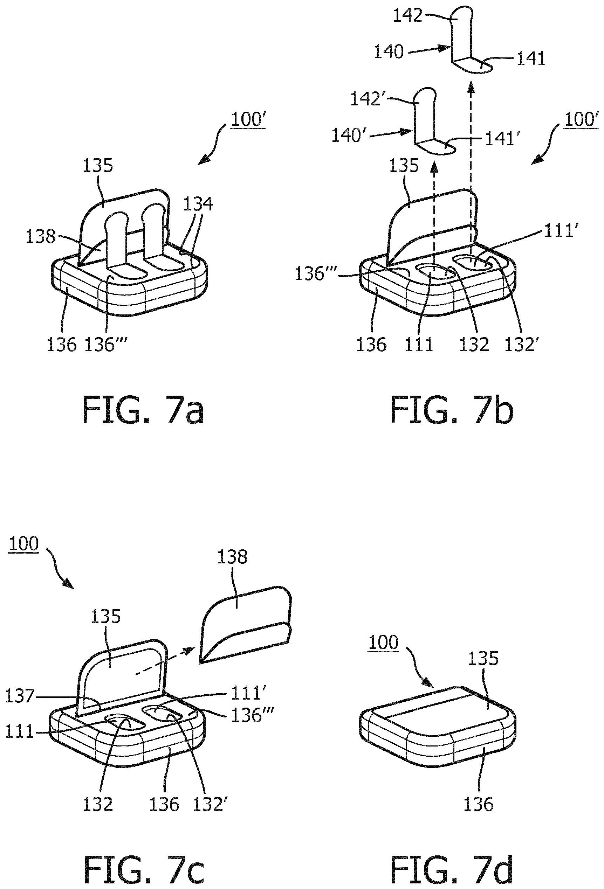

Fig. 7a, 7b, 7c and 7d illustrate a wearable sensing device 100' before use and a wearable sensing device 100 in operation or ready to use, comprising two air- or oxygene dependent batteries (not shown here), and method to prepare the wearable sensing device 100 before use. Similarly to the embodiments of Figs. 4 to 6, the housing 130 is made of two parts, a cover 135 and a base 136, watertightly monted one to the other once the sealing tabs 140-140' are removed. In this embodiment, at least a part of the cover 135 is arranged to be foldable on a top side 136'" of the base 136 provided with two apertures 132-132', to a fixed and watertight position. The foldable part of the cover 135 may be in particular a lid that can rotate around a rotation axis 137 with respect to the base 136 so as to cover the apertures 132-132' and a top side 136'" of the base 136. As an option this top side 136'" of the base 136 can be formed with a recess having a shape complementary to the shape of the cover 135, improving then the interfacing between the two (at the interface 134). The fixed and watertight montage of the cover 135 to the base 136 may be performed at least partly via adhesive material provided on the inner part of the cover 135, and protected against environment before use thanks to a protective layer 138. Some additional attachment means and/or sealing elements may be alternatively or further provided at the interface 134. As an advantageous configuration the extended tabs 142-142' of the sealing tabs 140-140' extend in similar direction as the cover 135 in an unfolded position (as indicated in Fig. 6a and 6b) which may offer a guidance to the user for an easier way to remove the sealing tabs 140-140'. Furthermore, some casing elements (not shown), other than the combination apertures 132-132' and cover 135, may also be provided to the housing 130 to lock the batteries in a secured way inside the housing 110, one (or more) semi-permeable membrane (not shown) may also be provided through the cover 136, and any other option as indicated in this document may also be provided to this particular embodiment.

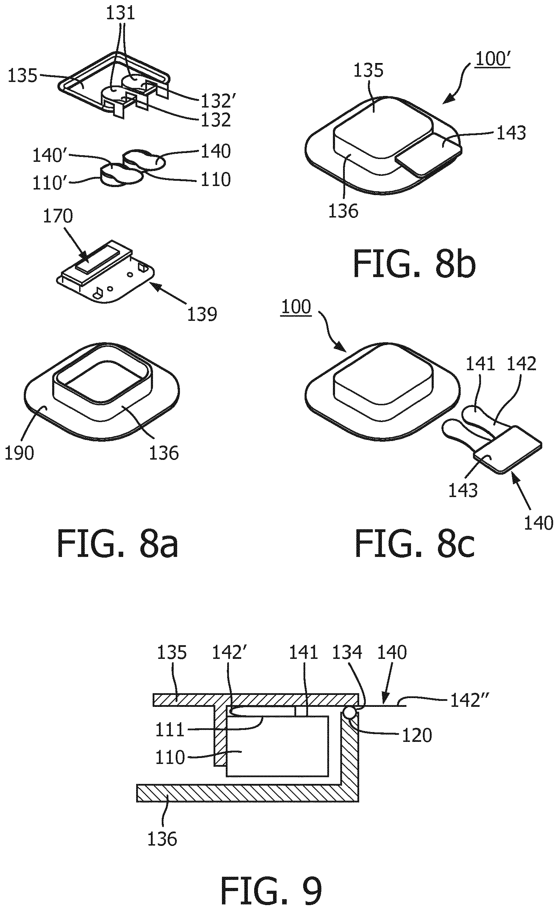

Fig. 8a, 8b, 8c and 9 illustrate a wearable sensing device 100' before use and a wearable sensing device 100 in operation or ready to use, comprising two air- or oxygene dependent batteries (not shown here), and method to prepare the wearable sensing device 100 before use. The housing is made of at least a cover 135 and a base 136, fixedly and watertightly monted one to the other before and once the sealing tabs 140-140' are removed. Optionally, the cover 135 or the base 136 can be made of two or more parts mounted one or the other. Fig.8a shows how the base 136 is monted to the cover 135, together with the inner components (insulating board 139 with electrical circuitry, including an antenna 170 for wireless communication, two batteries 110-110' provided with their sealing tabs 140-140') and the body interface 190. The cover 135 is provided with casing elements 131 arranged to fit with most of the sides of the batteries 110-110' such that the batteries 110-110' are locked in a locking position in the housing, optionally also via the board 139 and the apertures 132-132'. In a more particular embodiment (not illustrated), the cover 135 may be made of two parts wherein one part is monted to the base 136 according to a similar principle as the one illustrated by Fig. 8a, and another part is a foldable part 135 according to the embodiment depicted by Figs. 7a-7b. Now, in this embodiment, the cover 135 can be monted on the base 136, even if the sealing tabs 140-140' are still in place thanks to the embodiment depicted by Fig. 9. In this particular embodiment of Fig. 9, a first part 142' of each extended tab 142 of each sealing tab 140 is folded in the housing, between the base 136 and the cover 135, and a second part 142" of the extended tab 142 extends beyond the housing through a slot 134 provided in the housing to this purpose (this slot 134 being in fact an interface or an opening between the cover 135 and the base 136 in this illustrated case). The folding of the extended tab 142 results in an increase of the length of the sealing tab 130, which helps for its removal, and allows a peeling force to be exerted on the sealing element 141 away from the air-inlet surface 111 of each battery 140-140' - i.e. this force comprises a non-neglictible component normal to this surface 111 - which would not be the same if this extended tab 142 would have not be folded - in this last case, the force exerted on the sealing element 141 would have been a sheering force (i.e. force in a direction along the surface 111) which would have led to a much more difficult sealing tab 140 removal. This embodiment allows then having a non-cumbersome and easy to use wearable sensing device 100, with sealing tabs 140-140' being easy to remove. To further facilitate the removal of the sealing tabs 140-140', a common tongue 143 (joining the end parts of the extended tabs 142-142') may be provided. This last optional improvement may be used also in the previous embodiments disclosed in this document. Preferably, a flexible sealing component 120 is further provided in the slot 134 to operate a watertight sealing after the sealing tabs 140-140' have been removed - this flexible sealing component 120 being typically in a constraint state before the sealing tabs 140-140' are removed which is at least partly released after sealing tabs 140-140' are removed - "seal-pulling force". Furthermore, some casing elements (not shown), other than the combination apertures 132-132'- casing elements 131 - cover 135, may also be provided to the housing 130 to lock the batteries in a secured way inside the housing 110, one (or more) semi-permeable membrane (not shown) may also be provided through the cover 135, and any other option as disclosed in this document may also be provided to this particular embodiment. Alternatively the casing elements 131 (and apertures 132-132') maybe provided in the base 136, or: partly in the base 136 and partly in the cover 135.

The wearable sensing device 100 as disclosed in this document may be configured to be worn by a subject at any location on the body of the subject. In some embodiments, the location may be a location comprising skin surface on organs or close to a bone (e.g. to ensure a well-defined contact with the skin). For example, in some embodiments, the device can be configured to be worn on a chest, a belly, on a back, an arm (upper or lower) of the subject, a wrist or finger of a subject, a forehead of a subject, or any other location on the body of the subject suitable for the application.

The wearable sensing device 100 may be especially nice to use with accelerometer as a sensing element 180, since it does not require special interface 190 with the user body and the wearable sensing device 100 is light, easy to use in a safe way in all types of environment for a long time. It may further be wireless and used in a lot of applications (health monitoring at home or in professional sites - nursing or clinical sites, fitness and/or coaching of physical activities, etc.).

The subject can be any type of subject (e.g. a patient or any other subject). The device can be for use by a trained user, such as a medical professional (e.g. a doctor, a nurse, or any other medical professional) or an untrained user (e.g. the subject themselves, a care giver, a family member, or any other untrained user). The device can be for use in a medical environment (e.g. a hospital, a surgery, or any other medical environment) and/or non-medical environment (e.g. at home, or any other non-medical environment).

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. Wearable sensing device (100) comprising:
one sensing element (180) to sense physiological signs of a user wearing the device;
electrical circuitry (170-172) to process signals from the sensing element (180);
at least one air- or oxygen-dependent battery (110) adapted to power the sensing element (180) and the circuitry (170-172), comprising an air-inlet surface (111);
a housing (130) of the sensing element (180), the circuitry (170-172) and the at least one battery (110) which are functionally electrically arranged together;
wherein the housing (130) comprises:
casing elements (131, 131') arranged to lock the at least one battery (110) in a predetermined locking position;
a base (136); and
a cover (135) mounted to the base (136) in a watertight manner and in such a way that an internal gap (132) extends between the air-inlet surface (111) of at least one battery (110) and the cover (135), the internal gap being formed by an aperture (132) initially provided in the cover (135) and/or in the base (136) before montage;
wherein the base (136) and the cover (135) are further arranged such that said air-inlet surface (111) has an access to ambient air.

2. Wearable sensing device (100') comprising:
one sensing element (180) to sense physiological signs of a user wearing the device;
electrical circuitry (170-172) to process signals from the sensing element (180);
at least one air- or oxygen-dependent battery (110) adapted to power the sensing element (180) and the circuitry (170-172), comprising an air-inlet surface (111);
a housing (130) of the sensing element (180), the circuitry (170-172) and the at least one battery (110) which are functionally electrically arranged together;
a sealing tab (140) comprising a battery sealing element (141) which air-tightly covers directly the air-inlet surface (111) of at least one battery (110), and an extended tab (142) extending from the sealing element (141) and adapted to be manually actionable; wherein the housing (130) comprises casing elements (131, 131') arranged to lock the at least one battery (110) in a predetermined locking position; and
wherein the housing (130) and the sealing tab (140) are arranged such that the sealing tab (140) is manually removed by peeling it away from the air-inlet surface (111) of the battery (110) and the housing (130) is adapted to have a watertight configuration after the removal of the sealing tab (140).

3. Wearable sensing device (100') of the preceding claim, wherein a first part of the extended tab (142') of at least one sealing tab (140) is folded in the housing (130) and a second part of the extended tab (142") extends beyond the housing (130) through an opening (134) of the housing (130).

4. Wearable sensing device (100') of the preceding claim, wherein a flexible sealing component (120) is provided in the opening (134) to provide a watertight sealing after the sealing tab (140) is removed.

5. Wearable sensing device (100, 100') of any of the preceding claims, comprising a plurality of batteries (110, 110') and a plurality of sealing tabs (140, 140'), and wherein the sealing tabs (140, 140') are provided with a common tongue (143) extending outside the housing (130).

6. Wearable sensing device (100') of claim 2 to 4, wherein the housing (130) comprises a base (136) and a cover (135), the cover (135) being mounted or to be mounted to the base (136) in a watertight manner, the cover (135) and/or the base (136) being provided with an aperture (132) that will form an internal gap extending between the air-inlet surface (111) of at least one battery (110) and the cover (135) once the sealing tab (140) is removed and the cover (135) is mounted to the base (136), and optionally the sealing element (141) extends on the air-inlet surface (111) of the battery (110) across or beneath the aperture (132).

7. Wearable sensing device (100') of any of the claims 1 or 6, wherein the aperture or the internal gap (132) facing the air-inlet surface (111) of a battery (110) is sufficiently narrow in at least one direction to prevent the corresponding battery (110) to leave out the housing (130).

8. Wearable sensing device (100, 100') of claim 1 or any of claims 6 and 7, wherein the cover (135) comprises a semi-permeable membrane (133) being water-tight and air-permeable.

9. Wearable sensing device (100, 100') of claim 1 or any of claims 6 and 7, wherein the cover (135) is arranged to be slid onto the base (136) to a fixed and watertight position, and wherein the base (136) and/or the cover (135) further comprise attachment elements to secure an attachment of the cover (135) to the base (136) at the fixed and watertight position.

10. Wearable sensing device (100, 100') of claim 1 or any of claims 6 and 7, wherein the cover (135) is arranged to be foldable on a top side (136'") of the base (136) provided with the at least one aperture (132, 132') to a fixed and watertight position, and wherein the base (136) and/or the cover (135) further comprise attaching elements to secure an attachment of the cover (135) to the base (136) at the fixed and watertight position.

11. Wearable sensing device (100') of claims 2 and 10, wherein the extended tab (142) of at least one sealing tab (140) is at least partly folded when the cover (135) is in an unfolded position.

12. Wearable sensing device (100, 100') of claim 1 or any of claims 6 and 7, wherein at least one portion of the housing (130) provided on edges of the aperture (132) is arranged to be elastically deformable to allow the battery (110) to be positioned into the housing (130).

13. Wearable sensing device (100, 100') of any of the preceding claims, wherein the casing elements (131, 131') of the housing (130) are provided with inner opposite walls fitting to at least one battery such that opposite sides of the battery (110) are sufficiently pressed by opposite inner walls to hold the battery (110) in the housing (130).

14. Wearable sensing device of any of the preceding claims, further comprising a communication circuitry arranged to wirelessly communicate sensing data, optionally processed by the electrical circuitry, to a separated receiver, and optionally to further wirelessly receive from a separated transmitter, parameters or instructions for tuning or controlling the wearable sensing device.

15. Wearable sensing device of any of the preceding claims, the casing elements of the housing comprises a circuit board on which the electrical circuitry is provided, the circuit board being positioned at an inner bottom side of the housing, and wherein the at least one battery is positioned on and electrically connected to the PCB and extending between the inner bottom side and the top side of the housing.
